# EUROPÄISCHE PATENTSCHRIFT

(11) **EP 3 651 729 B1**
(45) Veröffentlichungstag und Bekanntmachung des Hinweises auf die Patenterteilung: **29.11.2023**
(21) Anmeldenummer: 18736886.5
(22) Anmeldetag: 02.07.2018
(51) Int. Cl.: A61K 8/34, A61K 8/81, A61Q 5/06, A61K 8/19, A61K 8/04, A61Q 1/02, A61Q 1/10

(54) **WASSERBESTÄNDIGE KOSMETISCHE MITTEL ZUR TEMPORÄREN FARBVERÄNDERUNG VON KERATINHALTIGEN MATERIALIEN I**
WATER-RESISTANT COSMETIC MEANS FOR TEMPORARILY CHANGING THE COLOUR OF KERATIN-CONTAINING MATERIALS I
PRODUITS COSMÉTIQUES RÉSISTANT À L'EAU POUR LA MODIFICATION TEMPORAIRE DE LA COULEUR DE MATIÈRES KÉRATINIQUES

(30) Priorität: 11.07.2017 DE 102017211853
(43) Veröffentlichungstag der Anmeldung: 20.05.2020
(73) Patentinhaber: Henkel AG & Co. KGaA, 40589 Düsseldorf (DE)
(72) Erfinder: GOUTSIS, Konstantin, 41363 Jüchen (DE); KESSLER-BECKER, Daniela, 51371 Leverkusen (DE)
(86) Internationale Anmeldenummer: PCT/EP2018/067817
(87) Internationale Veröffentlichungsnummer: WO 2019/011697

(56) Entgegenhaltungen:
- DE-A1-102005 028 388
- DE-A1-102015 204 154
- DATABASE GNPD [Online] MINTEL; 1. Juni 2017 (2017-06-01), "Black Platinum Hair Styling Gel", XP002784118, Database accession no. 4903517
- DATABASE GNPD [Online] MINTEL; 1. Juli 2010 (2010-07-01), "Glitter Hair Make-up", XP002784119, Database accession no. 1363825
- DATABASE GNPD [Online] MINTEL; 1. Juni 2017 (2017-06-01), "Fashion Hair Spray", XP002784120, Database accession no. 4874893
- DATABASE GNPD [Online] MINTEL; 1. November 2007 (2007-11-01), "Mascara", XP002784121, Database accession no. 814579
- DATABASE GNPD [Online] MINTEL; 1. Januar 2008 (2008-01-01), "Hot Mixing Glitter", XP002784122, Database accession no. 825975
- DATABASE GNPD MINTEL; 1. Januar 2008 (2008-01-01), "Silver Glitter Spray", XP002784123, Database accession no. 830780
- DATABASE GNPD [Online] MINTEL; 1. August 2012 (2012-08-01), "Fibre-Cream", XP002784124, Database accession no. 1875143
- DATABASE GNPD [Online] MINTEL; 1. Januar 2017 (2017-01-01), "Thrill Texture Fibre Gum", XP002784125, Database accession no. 4535503
- DATABASE GNPD MINTEL; 1. Januar 2017 (2017-01-01), "Wax", XP002784126, Database accession no. 4582085

## Beschreibung

Die Anmeldung betrifft das technische Gebiet der wasserbeständigen temporären Farbveränderung von keratinhaltigen Materialien, insbesondere menschlicher Haut und/oder menschlicher Haare. Gegenstand der Anmeldung sind kosmetische Mittel, enthaltend mindestens einen aliphatischen und/oder aromatischen Alkohol, mindestens ein anionisches Polymer auf Basis von Acrylsäure und Acrylaten sowie mindestens ein Pigment. Schließlich sind Gegenstand der vorliegenden Anmeldung Verfahren zur temporären Färbung keratinischer Fasern und der Haut unter Anwendung dieser Mittel sowie die Verwendung von speziellen anionischen Polymeren in Pigment-haltigen kosmetischen Mitteln zur Verbesserung der Wasserbeständigkeit dieser Mittel.

Die Veränderung der Farbe von keratinischen Oberflächen, insbesondere von menschlicher Haut und/oder Haaren, stellt einen wichtigen Bereich der modernen Kosmetik dar. Zur Veränderung der Haarfarbe kennt der Fachmann je nach Anforderung an die Färbung diverse Färbesysteme. Für permanente, intensive Färbungen mit guten Echtheitseigenschaften und guter Grauabdeckung werden Oxidationsfärbemittel verwendet. Solche Färbemittel enthalten üblicherweise Oxidationsfarbstoffvorprodukte, sogenannte Entwicklerkomponenten und Kupplerkomponenten, welche unter dem Einfluss von Oxidationsmitteln wie beispielsweise Wasserstoffperoxid, untereinander die eigentlichen Farbstoffe ausbilden. Oxidationsfärbemittel zeichnen sich durch sehr langanhaltende Färbeergebnisse aus.

Bei dem Einsatz von direktziehenden Farbstoffen diffundieren bereits fertig ausgebildete Farbstoffe aus dem Färbemittel in die Haarfaser hinein. Im Vergleich zur oxidativen Haarfärbung weisen die mit direktziehenden Farbstoffen erhaltenen Färbungen eine geringere Haltbarkeit und schnellere Auswaschbarkeit auf. Färbungen mit direktziehenden Farbstoffen verbleiben üblicherweise für einen Zeitraum zwischen 5 und 20 Haarwäschen auf dem Haar.

Zur Veränderung der Hautfarbe, insbesondere zur Bräunung der Haut oder zur Verminderung von unerwünschten Altersflecken, sind im Stand der Technik verschiedene Verfahren bekannt. Die Bräunung der Haut kann beispielsweise unter Verwendung von Farbstoffen erfolgen, welche in die Hautschichten eindringen und eine anhaltende Bräunung verursachen. Zur Verminderung von Altersflecken werden Wirkstoffe eingesetzt, welche in die Haut eindringen und die Altersflecken verursachenden Melanin-Ansammlungen zersetzen. Auch hier soll eine lang anhaltende Veränderung der Hautfarbe, welche sich nicht oder nur schwer durch Reinigung der Haut entfernen lässt, erreicht werden.

Im Rahmen moderner Modetrends besteht aber auch das Bedürfnis von Farbeffekten, welche lediglich für einen kurzen Zeitraum auf dem Haar und/oder der Haut verbleiben und sich im Anschluss daran durch eine einzige Wäsche mit tensidhaltigen Reinigungsmitteln völlig rückstandslos von den Haaren und/oder der Haut entfernen lassen. Bei Kontakt mit Wasser oder Schweiß soll der Farbeffekt hingegen erhalten bleiben, um ein Herablaufen der Farbe durch Umwelteinflüsse, wie Regen oder Schweiß, zu vermeiden. Direktziehende Farbstoffe diffundieren mehr oder weniger stark in die Haarfaser oder die Hautoberfläche hinein und überdauern dort mehrere Wäschen mit tensidhaltigen Reinigungsmitteln. Zur rückstandslosen Entfernung des Farbeffektes nach einmaliger Wäsche mit tensidhaltigen Reinigungsmitteln ist diese Farbstoffklasse daher nicht gut geeignet.

Für kurzzeitige Farbveränderungen auf dem Haar und/oder der Haut ist der Einsatz von Farbpigmenten bekannt. Unter Farbpigmenten werden im Allgemeinen unlösliche, farbgebende Substanzen verstanden. Diese liegen ungelöst in Form kleiner Partikel in der Färbeformulierung vor und lagern sich lediglich von außen auf den Haarfasern und/oder der Hautoberfläche ab. Daher lassen sie sich durch eine einzige Wäsche mit tensidhaltigen Reinigungsmitteln wieder rückstandslos entfernen. Unter dem Namen Haar-Mascara sind verschiedene Produkte dieses Typs auf dem Markt erhältlich.

Da die Entfernung der Haar-Mascaras durch eine Haarwäsche möglich ist, sind sie im Regelfall als "leave-on" Produkte konzipiert. Von besonderem Vorteil ist es für den Anwender eines "leaveon" Produktes, wenn er gleichzeitig mit der temporären Farbänderung auch eine leichte temporäre Formgebung der Haare vornehmen kann. Als temporäre Formgebungen kommen beispielsweise Gestaltungen wie Lockung, Glättung, Toupierung oder auch Festigung in Frage. Temporäre Formgebungen können beispielsweise durch Stylingmittel, wie Haarsprays, Haarwachse, Haargele, Haarfestiger, Fönwellen, Stylingsprays usw. erzielt werden. Das temporäre Formgeben wird auch als Hair-Styling oder Styling bezeichnet, Formgebungsmittel werden auch als Stylingmittel bezeichnet.

Produkte, welche eine temporäre Farb- und/oder Formänderung keratinischer Oberflächen erlauben, sind bereits aus dem Stand der Technik bekannt. Derartige Produkte enthalten zumeist eine Mischung aus filmbildenden Polymeren sowie Pigmenten. Jedoch weisen die Produkte keine ausreichende Wasserbeständigkeit auf.

Die Aufgabe der vorliegenden Erfindung bestand in der Bereitstellung eines vielfältig einsetzbaren Mascara Produktes, welches die temporäre Farbänderung von Haaren und/oder der Haut ermöglicht. Das Produkt sollte so konfektionierbar sein, dass es mittels eines Schwamms, einer Bürste oder einer Sprühapplikation aufgetragen werden kann. Die Farbänderung sollte hierbei einfach und schädigungsarm erfolgen und durch eine einzige Wäsche mit tensidhaltigen Reinigungsmitteln rückstandlos von den Haaren und/oder der Haut entfernbar sein. Bis zum Zeitraum der nächsten Wäsche sollte das auf der keratinischen Oberfläche befindliche Produkt gegenüber äußeren Einflüssen jedoch äußerst resistent sein, d.h. durch Einwirkung von Wasser oder Schweiß, Abrieb auf Textilien oder Kämmen sollte kein Farbverlust oder eine sonstige Ablösung des Produktes auftreten. Gleichzeitig sollte die auf diese Weise farbveränderte keratinische Oberfläche gute kosmetische Eigenschaften aufweisen.

Überaschenderweise hat sich gezeigt, dass diese Aufgaben durch Einsatz von Farbpigmenten und bestimmten anionischen Polymeren gelöst werden können, wenn diese in einem speziellen alkoholischen Träger eingesetzt werden. Der Einsatz derartiger Polymere führt zu einer hohen Wasserbeständigkeit der kosmetischen Mittel, ohne jedoch die kosmetischen Eigenschaften dieser Mittel negativ zu beeinflussen. Zudem lässt sich die Färbung rückstandsfrei mittels einer einzigen Wäsche mit tensidhaltigen Reinigungsmitteln entfernen und führt daher, im Gegensatz zu einer Färbung mit Oxidationsfarbstoffen und direktziehenden Farbstoffen oder der Bleiche mit Oxidationsmitteln, nur zu einer temporären Färbung der keratinischen Oberflächen, insbesondere der Haut und/oder der Haare.

Ein erster Gegenstand der vorliegenden Erfindung ist somit ein kosmetisches Mittel, enthaltend
a) mindestens einen aliphatischen und/oder aromatischen Alkohol mit 2 bis 8 Kohlenstoffatomen,
b) mindestens ein anionisches Polymer, umfassend mindestens eine Struktureinheit der Formel (I) und mindestens eine Struktureinheit der Formel (II) und mindestens eine Struktureinheit der Formel (III) 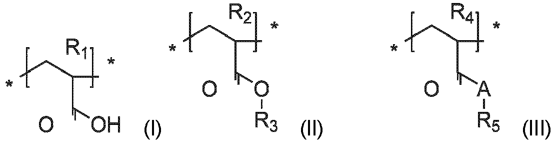 worin
   R₁, R₂ und R₄, jeweils unabhängig voneinander, für ein Wasserstoffatom oder eine C₁₋C₄- Alkylgruppe stehen,
   R₃ für eine verzweigte oder unverzweigte, gesättigte oder ungesättigte C₁-C₁₂-Alkygruppe steht,
   R₅ für eine verzweigte oder unverzweigte, gesättigte oder ungesättigte C₆-C₁₄-Alkylgruppe steht und
   A für Sauerstoff, Schwefel oder eine NH-Gruppe steht, und

a) 5,0 bis 20 Gew.-% mindestens eines Pigments, wobei
   das kosmetische Mittel als aliphatischen und/oder aromatischen Alkohol mit 2 bis 8 Kohlenstoffatomen a) - bezogen auf sein Gesamtgewicht -
   a1) 70 bis 90 Gew.-% Ethanol und
   a2) 1,2-Propandiol und/oder Glycerin in einer Gesamtmenge von 1,0 bis 3,5 Gew.-% enthält.

Gemäß obiger Formeln und aller folgenden Formeln steht eine chemische Bindung, welche mit dem Symbol "*" gekennzeichnet ist, für eine freie Valenz des entsprechenden Strukturfragments. Unter freier Valenz ist hierbei die Anzahl von Atombindungen zu verstehen, welche von dem entsprechenden Strukturfragment an der mit dem Symbol "*" gekennzeichneten Position ausgehen. Im Rahmen der vorliegenden Erfindung geht bevorzugt jeweils eine Atombindung von den mit dem Symbol "*" gekennzeichneten Positionen der Strukturfragmente zu weiteren Strukturfragmenten aus.

Im Rahmen der vorliegenden Erfindung werden unter dem Begriff "anionische Polymere" solche Polymere verstanden, welche in einem protischen Lösemittel bei Standardbedingungen mindestens eine Struktureinheit mit permanent anionischen Gruppen tragen, wobei die anionischen Gruppen durch Gegenionen unter Erhaltung der Elektroneutralität kompensiert werden müssen. Unter anionische Gruppen fallen erfindungsgemäß insbesondere Carboxylgruppen.

Die Angabe Gew.-% bezieht sich vorliegend, sofern nichts anderes angegeben ist, auf das Gesamtgewicht der erfindungsgemäßen kosmetischen Mittel, wobei die Summe aller Inhaltsstoffe der erfindungsgemäßen Mittel 100 Gew.-% ergibt. Enthalten die erfindungsgemäßen kosmetischen Mittel mindestens ein Treibmittel, so bezieht sich die Angabe Gew.-% auf das Gesamtgewicht des kosmetischen Mittels inklusive des vorhandenen Treibmittels.

Als ersten wesentlichen Bestandteil a) enthält das erfindungsgemäße kosmetische Mittel mindestens einen aliphatischen und/oder aromatischen Alkohol mit 2 bis 8 Kohlenstoffatomen, wobei a) - bezogen auf sein Gesamtgewicht -
a1) 70 bis 90 Gew.-% Ethanol und
a2) 1,2-Propandiol und/oder Glycerin in einer Gesamtmenge von 1,0 bis 3,5 Gew.-% enthält.

Als zweiten wesentlichen Bestandteil b) enthält das erfindungsgemäße kosmetische Mittel mindestens ein anionisches Polymer, welches mindestens eine Struktureinheit der Formeln (I) bis (III) enthält. In den Struktureinheiten der Formeln (I) bis (III) können die Reste R1, R2 und R4 für C1- C4-Alkylgruppen stehen. Beispiele für derartige Gruppen sind Methyl-, Ethyl-, Propyl-, Isopropyl-, Hydroxpropyl, Butyl-, Sec-Butyl-, Isobutyl-, tert-Butyl- sowie Hydroxybutylgruppen. Weiterhin können die Reste R₃ und R₅ in den Struktureinheiten der Formeln (II) und (III) für C₁-C₁₂- bzw. C₆-C₁₄-Alkylgruppen stehen. Derartige Gruppen sind beispielsweise Pentyl-, Hexyl-, Heptyl-, Capryl-, Caprin-, Lauryl- und Myristylgruppen.

Im Rahmen der vorliegenden Erfindung ist es bevorzugt, wenn die Reste R₁, R₂ und R₄ in den Formeln (I) bis (III) für bestimmte Gruppen stehen. Bevorzugte Ausführungsformen dieses Erfindungsgegenstandes sind daher dadurch gekennzeichnet, dass in den Struktureinheiten der Formeln (I) und (III) die Reste R₁ und R₄, jeweils unabhängig voneinander, für ein Wasserstoffatom stehen und dass in der Struktureinheit der Formel (II) der Rest R₂ für eine Methylgruppe steht. Bevorzugte werden daher anionischen Polymere auf Basis von Acrylsäure, Methacrylaten und Acrylamiden bzw. Acrylaten eingesetzt. Der Einsatz derartiger anionischer Polymere führt zu einer besonders hohen Wasserbeständigkeit der erfindungsgemäßen kosmetischen Mittel.

Weiterhin hat es sich im Rahmen der vorliegenden Erfindung als vorteilhaft erwiesen, wenn der Rest R₃ in der Struktureinheit der Formel (II) für bestimmte Gruppen steht. Es ist daher bevorzugt, dass in der Struktureinheit der Formel (II) der Rest R₃ für eine verzweigte C₃-C₆-Alkylgruppe, insbesondere für eine *-CH₂-C(CH₃)₂-Gruppe, steht. Hierbei zeigt das *-Symbol die Verknüpfung des Restes R₃ mit dem Sauerstoffatom der Struktureinheit der Formel (II) an. Der Rest R₃ ist also über die CH₂-Gruppe an die Carbonylgruppe der Struktureinheit der Formel (II) gebunden. Der Einsatz von anionischen Polymeren, welche insbesondere verzweigte Methacrylate enthalten, hat sich als besonders vorteilhaft im Hinblick auf die Wasserbeständigkeit einerseits und die Auswaschbarkeit der Pigmente mittels tensidhaltiger Reinigungsmittel andererseits herausgestellt.

Weiterhin ist es erfindungsgemäß bevorzugt, wenn in der Struktureinheit der Formel (III) A für eine NH-Gruppe steht. Bevorzugte anionische Polymere enthalten daher mindestens eine Struktureinheit auf Basis von Acrylamiden. Der Einsatz von anionischen Polymeren auf Basis von Acrylamiden führt zu einer verbesserten Resistenz gegen äußere Umwelteinflüsse, ohne jedoch die Auswaschbarkeit mit tensidhaltigen Reinigungsmitteln negativ zu beeinflussen.

Zudem hat es sich im Rahmen der vorliegenden Erfindung als vorteilhaft erwiesen, wenn der Rest R₅ in der Struktureinheit der Formel (III) für bestimmte Gruppen steht. Es ist daher erfindungsgemäß bevorzugt, wenn in der Struktureinheit der Formel (III) der Rest R₅ für eine verzweigte C₆-C₁₀-Alklygruppe, insbesondere für eine *-C(CH₃)₂-CH₂-C(CH₃)₃-Gruppe, steht. Hierbei zeigt das *-Symbol die Verknüpfung des Restes R₅ mit der Einheit Ader Struktureinheit der Formel (III) an. Der Einsatz von anionischen Polymeren, welche insbesondere verzweigte Acrylamide und Acrylate enthalten, hat sich als besonders vorteilhaft im Hinblick auf die Wasserbeständigkeit einerseits und die Auswaschbarkeit der Pigmente mittels tensidhaltiger Reinigungsmittel andererseits herausgestellt.

Erfindungsgemäß besonders vorteilhaft werden demnach anionische Polymere eingesetzt, welche mindestens eine Struktureinheit der Formel (I), mindestens eine Struktureinheit der Formel (II) und mindestens eine Struktureinheit der Formel (III) enthalten 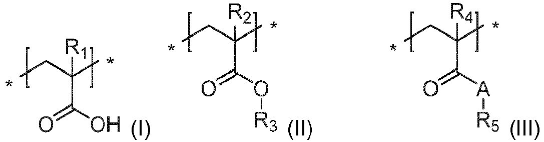 worin
R₁ und R₄, jeweils für ein Wasserstoffatom stehen,
R₂ für eine Methylgruppe steht,
R₃ für eine *-CH₂-C(CH₃)₂-Gruppe steht,
R₅ für eine *-C(CH₃)₂-CH₂-C(CH₃)₃-Gruppe steht und
A für eine NH-Gruppe steht.

Der Einsatz derartiger anionischer Polymere auf Basis von Acrylsäure, 2-Methylpropylmethacrylat sowie Octylacrylamid hat sich als besonders vorteilhaft im Hinblick auf die Beständigkeit gegen äußere Umwelteinflüsse einerseits sowie die Auswaschbarkeit der erfindungsgemäßen kosmetischen Mittel durch tensidhaltige Reinigungsmittel andererseits erwiesen. Daher resultiert der Einsatz derartiger Polymere in Pigment-haltigen kosmetischen Mittel in einer wasserbeständigen temporären Färbung, welche durch einmalige Anwendung von tensidhaltigen Reinigungsmitteln entfernt werden kann. Zudem lässt sich mit diesen Polymeren ein hoher und flexibler Frisurenhalt, welcher bei Einwirkung von Feuchtigkeit nicht verklebt und daher einen unnatürlichen Eindruck hinterlässt, erreichen.

Bevorzugt eingesetzte anionische Polymere weisen bestimmte mittlere Molekulargewichte M_{w} auf. Die Bestimmung dieser Molekulargewichte kann beispielsweise durch Kopplung einer Gelpermeationschromatographie (GPC) an ein Fourier-Transform-Massenspektrometer (FTMS) wie in Aaserud D.J et. al; "Gel Permeation Chromatography Coupled to Fourier Transform Mass Spectrometry for Polymer Characterization"; Anal. Chem. 1999, 71, 4793-4799 beschrieben, erfolgen. Bevorzugte Ausführungsformen dieses Erfindungsgegenstands sind daher dadurch gekennzeichnet, dass das anionische Polymer ein mittleres Molekulargewicht M_{w} von 50.000 bis 250.000 g/mol, vorzugsweise von 80.000 bis 220.000 g/mol, bevorzugt von 100.000 bis 200.000 g/mol, insbesondere von 110.000 bis 180.000 g/mol, aufweist.

Bevorzugte erfindungsgemäße kosmetische Mittel enthalten das mindestens eine anionische Polymer a) in einer Gesamtmenge von 0,1 bis 10 Gew.-%, vorzugsweise von 0,5 bis 8,0 Gew.-%, bevorzugt von 1,0 bis 5,5 Gew.-%, insbesondere von 1,0 bis 2,5 Gew.-%, bezogen auf das Gesamtgewicht des kosmetischen Mittels. Die Verwendung dieser Mengen des anionischen Polymers resultiert in einer hohen Beständigkeit gegenüber äußeren Umwelteinflüssen, ohne jedoch die Anwendbarkeit dieser Mittel sowie die Lagerstabilität negativ zu beeinflussen. Bei Einsatz dieser Polymermengen in Mitteln zur gleichzeitigen temporären Farb- und Formveränderung der Haare wird neben einer wasserbeständigen Färbung auch ein hoher und flexibler Frisurenhalt ermöglicht.

Als dritten wesentlichen Bestandteil c) enthält das erfindungsgemäße kosmetische Mittel mindestens ein Pigment. Unter Pigmenten im Sinne der vorliegenden Erfindung werden farbgebende Verbindungen verstanden, welche bei 20 °C in Wasser eine Löslichkeit von weniger als 0,1 g/l besitzen. Die Wasserlöslichkeit kann beispielsweise mittels der nachfolgend beschriebenen Methode erfolgen: 0,1 g des Pigments werden in einem Becherglas abgewogen. Ein Rührfisch wird hinzugefügt. Dann wird mit destilliertem Wasser (20 °C) auf 1 I aufgefüllt. Es wird für eine Stunde gerührt. Sind in der Mischung nach diesem Zeitraum noch ungelöste Bestandteile des Pigments sichtbar, so liegt die Löslichkeit des Pigments unterhalb von 0,1 g/l.

Die erfindungsgemäßen kosmetischen Mittel sollen bevorzugt zu einer temporären Farbgebung in Form von "metallic" Effekten führen. Nicht unter die Definition des Farbpigments fallen daher die Weißpigmente. Weißpigmente sind unbunte anorganische Pigmente mit einem hohen Brechungsindex (i.d.R größer 1,8), welche meist synthetisch hergestellt und vor allem zur Erzeugung von optischer Weiße in Anstrichmitteln oder als Füllstoff, z. B. in Kunststoffen, verwendet werden. Weißpigmente wie beispielsweise Titandioxid oder Zinkdioxid sind daher von der Definition des Farbpigments explizit nicht mit umfasst.

In den Mitteln liegen die Farbpigmente in Form von kleinen ungelösten Partikeln vor, welche nicht in die keratinische Oberfläche hineindiffundieren, sondern welche sich in dem durch die Komponente b) gebildeten Polymerfilms auf der Außenseite der keratinischen Oberfläche anlagern.

Geeignete Farbpigmente können organischen und/oder anorganischen Ursprungs sein. Bevorzugte Farbpigmente sind ausgewählt aus synthetischen oder natürlichen anorganischen Pigmenten. Anorganische Farbpigmente natürlichen Ursprungs können beispielsweise aus Kreide, Ocker, Umbra, Grünerde, gebranntem Terra di Siena oder Graphit hergestellt werden. Weiterhin können als anorganische Farbpigmente Schwarzpigmente wie z. B. Eisenoxidschwarz, Buntpigmente wie z. B. Ultramarin oder Eisenoxidrot sowie Fluoreszenz- oder Phosphoreszenzpigmente eingesetzt werden.

Bevorzugte Ausführungsformen des ersten Erfindungsgegenstands sind daher dadurch gekennzeichnet, dass das kosmetische Mittel als Farbpigment (b) mindestens ein anorganisches Farbpigment enthält, welches ausgewählt ist aus (i) farbigen Metalloxiden, (ii) Metallhydroxiden, (iii) Metalloxidhydraten, (iv) Silicaten, (v) Metallsufiden, (vi) komplexen Metallcyaniden, (vii) Metallsulfaten, (viii) Bronzepigmenten und/oder aus (ix) farbigen Pigmenten auf Mica- oder Glimmerbasis, die mit mindestens einem Metalloxid und/oder einem Metalloxychlorid beschichtet sind, sowie (x) deren Mischungen.

Besonders geeignet sind farbige Metalloxide, -hydroxide und -oxidhydrate, Mischphasenpigmente, schwefelhaltige Silicate, Silicate, Metallsulfide, komplexe Metallcyanide, Metallsulfate, -chromate und/oder -molybdate. Insbesondere bevorzugte Farbpigmente sind schwarzes Eisenoxid (CI 77499), gelbes Eisenoxid (CI 77492), rotes und braunes Eisenoxid (CI 77491), Manganviolett (CI 77742), Ultramarine (Natrium-Aluminiumsulfosilikate, CI 77007, Pigment Blue 29), Chromoxidhydrat (CI77289), Eisenblau (Ferric Ferrocyanide, CI77510) und/oder Carmine (Cochineal).

Erfindungsgemäß besonders bevorzugte Farbpigmente sind farbige Perlglanzpigmente. Diese basieren üblicherweise auf Mica- und/oder Glimmerbasis und können mit einem oder mehreren Metalloxiden beschichtet sein. Glimmer gehört zu den Schicht-Silicaten. Die wichtigsten Vertreter dieser Silicate sind Muscovit, Phlogopit, Paragonit, Biotit, Lepidolith und Margarit. Zur Herstellung der Perlglanzpigmente in Verbindung mit Metalloxiden wird der Glimmer, überwiegend Muscovit oder Phlogopit, mit einem Metalloxid beschichtet.

Alternativ zu natürlichem Glimmer kann auch ggfs. mit einem oder mehrere Metalloxide(en) beschichtetes, synthetisches Mica als Perlglanzpigment verwendet werden. Besonders bevorzugte Perlglanzpigmente basieren auf natürlichem oder synthetischem Mica (Glimmer) und sind mit einem oder mehreren der zuvor genannten Metalloxide beschichtet. Die Farbe der jeweiligen Pigmente kann durch Variation der Schichtdicke des oder der Metalloxids(e) variiert werden.

Es ist daher erfindungsgemäß besonders bevorzugt, wenn das mindestens eine Pigment ein farbiges Pigment auf Mica- oder Glimmerbasis ist, welches mit einem oder mehreren Metalloxiden aus der Gruppe aus Titandioxid (CI 77891), schwarzem Eisenoxid (CI 77499), gelbem Eisenoxid (CI 77492), rotem und/oder braunem Eisenoxid (CI 77491, CI 77499), Manganviolett (CI 77742), Ultramarine (Natrium-Aluminiumsulfosilikate, CI 77007, Pigment Blue 29), Chromoxidhydrat (CI 77289), Chromoxid (CI 77288) und/oder Eisenblau (Ferric Ferrocyanide, CI 77510) beschichtet ist.

Beispiele für besonders geeignete Farbpigmente sind im Handel beispielsweise unter den Handelsbezeichnungen Rona^{®}, Colorona^{®}, Xirona^{®}, Dichrona^{®} und Timiron^{®} von der Firma Merck, Ariabel^{®} und Unipure^{®} von der Firma Sensient, Prestige^{®} von der Firma Eckart Cosmetic Colors und Sunshine^{®} von der Firma Sunstar erhältlich.

Ganz besonders bevorzugte Farbpigmente mit der Handelsbezeichnung Colorona^{®} sind beispielsweise:
Colorona Copper, Merck, MICA, CI 77491 (IRON OXIDES)
Colorona Passion Orange, Merck, Mica, CI 77491 (Iron Oxides), Alumina
Colorona Patina Silver, Merck, MICA, CI 77499 (IRON OXIDES), CI 77891 (TITANIUM DIOXIDE)
Colorona RY, Merck, CI 77891 (TITANIUM DIOXIDE), MICA, CI 75470 (CARMINE)
Colorona Oriental Beige, Merck, MICA, CI 77891 (TITANIUM DIOXIDE), CI 77491 (IRON OXIDES)
Colorona Dark Blue, Merck, MICA, TITANIUM DIOXIDE, FERRIC FERROCYANIDE
Colorona Chameleon, Merck, CI 77491 (IRON OXIDES), MICA
Colorona Aborigine Amber, Merck, MICA, CI 77499 (IRON OXIDES), CI 77891 (TITANIUM DIOXIDE)
Colorona Blackstar Blue, Merck, CI 77499 (IRON OXIDES), MICA
Colorona Patagonian Purple, Merck, MICA, CI 77491 (IRON OXIDES), CI 77891 (TITANIUM DIOXIDE), CI 77510 (FERRIC FERROCYANIDE)
Colorona Red Brown, Merck, MICA, CI 77491 (IRON OXIDES), CI 77891 (TITANIUM DIOXIDE)
Colorona Russet, Merck, CI 77491 (TITANIUM DIOXIDE), MICA, CI 77891 (IRON OXIDES)
Colorona Imperial Red, Merck, MICA, TITANIUM DIOXIDE (CI 77891), D&C RED NO. 30 (CI 73360)
Colorona Majestic Green, Merck, CI 77891 (TITANIUM DIOXIDE), MICA, CI 77288 (CHROMIUM OXIDE GREENS)
Colorona Light Blue, Merck, MICA, TITANIUM DIOXIDE (CI 77891), FERRIC FERROCYANIDE (CI 77510)
Colorona Red Gold, Merck, MICA, CI 77891 (TITANIUM DIOXIDE), CI 77491 (IRON OXIDES)
Colorona Gold Plus MP 25, Merck, MICA, TITANIUM DIOXIDE (CI 77891), IRON OXIDES (CI 77491)
Colorona Carmine Red, Merck, MICA, TITANIUM DIOXIDE, CARMINE
Colorona Blackstar Green, Merck, MICA, CI 77499 (IRON OXIDES)
Colorona Bordeaux, Merck, MICA, CI 77491 (IRON OXIDES)
Colorona Bronze, Merck, MICA, CI 77491 (IRON OXIDES)
Colorona Bronze Fine, Merck, MICA, CI 77491 (IRON OXIDES)
Colorona Fine Gold MP 20, Merck, MICA, CI 77891 (TITANIUM DIOXIDE), CI 77491 (IRON OXIDES)
Colorona Sienna Fine, Merck, CI 77491 (IRON OXIDES), MICA
Colorona Sienna, Merck, MICA, CI 77491 (IRON OXIDES)
Colorona Precious Gold, Merck, Mica, CI 77891 (Titanium dioxide), Silica, CI 77491(lron oxides), Tin oxide
Colorona Sun Gold Sparkle MP 29, Merck, MICA, TITANIUM DIOXIDE, IRON OXIDES, MICA, CI 77891, CI 77491 (EU)
Colorona Mica Black, Merck, CI 77499 (Iron oxides), Mica, C! 77891 (Titanium dioxide)
Colorona Bright Gold, Merck, Mica, CI 77891 (Titanium dioxide), CI 77491 (Iron oxides)
Colorona Blackstar Gold, Merck, MICA, CI 77499 (IRON OXIDES)

Weiterhin besonders bevorzugte Farbpigmente mit der Handelsbezeichnung Xirona^{®} sind beispielsweise:
Xirona Golden Sky, Merck, Silica, CI 77891 (Titanium Dioxide), Tin Oxide
Xirona Caribbean Blue, Merck, Mica, CI 77891 (Titanium Dioxide), Silica, Tin Oxide
Xirona Kiwi Rose, Merck, Silica, CI 77891 (Titanium Dioxide), Tin Oxide
Xirona Magic Mauve, Merck, Silica, CI 77891 (Titanium Dioxide), Tin Oxide.

Zudem sind besonders bevorzugte Farbpigmente mit der Handelsbezeichnung Unipure^{®} beispielsweise:
Unipure Red LC 381 EM, Sensient CI 77491 (Iron Oxides), Silica
Unipure Black LC 989 EM, Sensient, CI 77499 (Iron Oxides), Silica
Unipure Yellow LC 182 EM, Sensient, CI 77492 (Iron Oxides), Silica

Für einige Anwendungsformen sind zudem o.g. Farbpigmente, deren Oberfläche hydrophobiert wurde, besonders geeignet. Es wurde gefunden, dass diese Pigmente insbesondere bei der Lagerung unter extremen Bedingungen mitunter vorteilhaft sein können.

Als Hydrophobierungsmittel kommen beispielsweise Silikone in Frage.

Hydrophob oberflächenbehandelte Farbpigmente werden beispielsweise in DE 102009051171 beschrieben, auf deren Inhalt an dieser Stelle ausdrücklich Bezug genommen wird.

Aufgrund ihrer ausgezeichneten Licht-, Wetter- und/oder Temperaturbeständigkeit ist die Verwendung der zuvor genannten anorganischen Farbpigmente in dem erfindungsgemäßen Mitteln besonders bevorzugt. Weiterhin ist es bevorzugt, wenn die eingesetzten Pigmente eine bestimmte Teilchengröße aufweisen. Diese Teilchengröße führt einerseits zu einer gleichmäßigen Verteilung der Pigmente in dem gebildeten Polymerfilm und vermeidet andererseits ein raues Haar- oder Hautgefühl nach dem Auftragen des kosmetischen Mittels. Es ist daher erfindungsgemäß vorteilhaft, wenn das mindestens eine Pigment eine mittlere Teilchengröße D₅₀ von 1,0 bis 50 µm, vorzugsweise von 5,0 bis 45 µm, bevorzugt von 10 bis 40 µm, insbesondere von 14 bis 30 µm, aufweist. Die mittlere Teilchengröße D₅₀ kann beispielsweise unter Verwendung von dynamischer Lichtstreuung (DLS) bestimmt werden.

Abhängig davon, welche Farbveränderung auf der keratinischen Oberfläche erwünscht wird, kann das mindestens eine Farbpigment c) in unterschiedlichen Mengen eingesetzt werden. Je mehr Farbpigment eingesetzt wird, desto höher ist generell das Ausmaß der Farbveränderung. Ab einer bestimmten Einsatzmenge läuft jedoch die Haftung der Pigmente an der Keratinfaser gegen einen Grenzwert, ab welchem es nicht mehr möglich ist, das Ausmaß der Farbveränderung durch weitere Erhöhung der eingesetzten Pigmentmenge zu steigern.

Das mindestens eine Pigment wird in einer Gesamtmenge von 5,0 bis 20,0 Gew.-%, bevorzugt von 7,0 bis 18,0 Gew.-%, insbesondere von 8,5 bis 15,5 Gew.-%, jeweils bezogen auf das Gesamtgewicht des kosmetischen Mittels, eingesetzt.

In Bezug auf die Wasserbeständigkeit des Pigment-haltigen Polymerfilms hat es sich als vorteilhaft herausgestellt, wenn ein bestimmtes Gewichtsverhältnis von Polymer b) zu Pigment c) in dem kosmetischen Mittel enthalten ist. Es ist daher erfindungsgemäß bevorzugt, wenn das kosmetische Mittel ein Gewichtsverhältnis des mindestens einen anionischen Polymers b) zu dem mindestens einen Pigment c) von 1,0 bis 6,0, vorzugsweise von 2,0 bis 5,5, bevorzugt von 2,5 bis 5,0, insbesondere von 3,0 bis 4,5, aufweist. Das zuvor angeführte Gewichtsverhältnis bezieht sich hierbei auf die Gesamtmengen an Polymer b) sowie Pigment c). Wird daher eine Mischung von verschiedenen Polymeren b) und/oder Pigmenten c) verwendet, so wird für die Berechnung des Gewichtsverhältnisses jeweils die Gesamtmenge der Mischung an Polymer b) und/oder Pigment c) herangezogen.

Eine besonders bevorzugte Ausführungsform im Rahmen des ersten Erfindungsgegenstands sind daher kosmetische Mittel zur temporären Verformung keratinischer Fasern, enthaltend
a) mindestens einen aliphatischen Alkohol mit 2 bis 8 Kohlenstoffatomen,
b) mindestens ein anionisches Polymer, umfassend mindestens eine Struktureinheit der Formel (I) und mindestens eine Struktureinheit der Formel (II) und mindestens eine Struktureinheit der Formel (III) 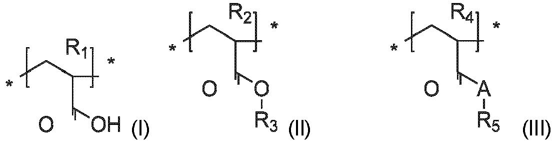 worin
   R₁ und R₄, jeweils für ein Wasserstoffatom stehen, R₂ für eine Methylgruppe steht,
   R₃ für eine *-CH₂-C(CH₃)₂-Gruppe steht,
   R₅ für eine *-C(CH₃)₂-CH₂-C(CH₃)₃-Gruppe steht und A für eine NH-Gruppe steht.
c) 5,0 bis 20 Gew.-% mindestens eines Pigments,
   wobei das kosmetische Mittel als aliphatischen Alkohol mit 2 bis 8 Kohlenstoffatomen a) - bezogen auf sein Gesamtgewicht -
   a1) 70 bis 90 Gew.-% Ethanol und
   a2) 1,2-Propandiol und/oder Glycerin in einer Gesamtmenge von 1,0 bis 3,5 Gew.-% enthält und
wobei c) ausgewählt ist aus farbigen Pigmenten auf Mica- oder Glimmerbasis, welche mit einem oder mehreren Metalloxiden aus der Gruppe aus Titandioxid (CI 77891), schwarzem Eisenoxid (CI 77499), gelbem Eisenoxid (CI 77492), rotem und/oder braunem Eisenoxid (CI 77491, CI 77499), Manganviolett (CI 77742), Ultramarine (Natrium-Aluminiumsulfosilikate, CI 77007, Pigment Blue 29), Chromoxidhydrat (CI 77289), Chromoxid (CI 77288) und/oder Eisenblau (Ferric Ferrocyanide, CI 77510) beschichtet sind.

Derartige kosmetische Mittel weisen eine hohe Beständigkeit gegen äußere Umwelteinflüsse, wie Regen, Schweiß, Abrieb durch Textilien und Kämmen, auf und lassen sich dennoch durch einmalige Anwendung von tensidhaltigen Reinigungsmitteln rückstandsfrei entfernen. Bei Auftragung auf keratinische Fasern, insbesondere Haare, wird zudem ein hervorragender Haltegrad sowie eine hohe Feuchteresistenz erreicht. Die hohe Feuchtigkeitsresistenz vermeidet hierbei ein Verkleben der Haare bei Einwirkung von Feuchtigkeit. Bei Anwendung auf der Haut weisen diese Mittel gute kosmetische Eigenschaften auf und führen nicht zu trockener oder fettiger Haut.

Die erfindungsgemäßen Mittel enthalten alle wesentlichen Bestandteile in einem Träger. Als Träger können insbesondere wässrige, wässrig-alkoholische und alkoholische Träger verwendet werden. Bei Einsatz von alkoholischen Trägern bildet der zuvor angeführte wesentliche Bestandteil a) den kosmetischen Träger. Durch den Wassergehalt des Trägers lasst sich die Ablagerung der Pigmente auf den keratinischen Oberflächen und die Filmbildung des Polymers b) ebenfalls beeinflussen. Ist der Wassergehalt zu hoch, besteht die Gefahr, dass das Produkt nicht ausreichend schnell trocknet. Insbesondere wenn die Mittel auf eine niedrigere Viskosität eingestellt werden (weil sie beispielsweise versprüht werden sollen), kann dass das Farbergebnis ungleichmäßig ausfallen. Es ist daher erfindungsgemäß bevorzugt, wenn das kosmetische Mittel Wasser in einer Gesamtmenge von 0 bis 30 Gew.-%, vorzugsweise von 0 bis 20 Gew.-%, bevorzugt von 0 bis 10 Gew.-%, insbesondere von 0 bis 2,0 Gew.-%, bezogen auf das Gesamtgewicht des kosmetischen Mittels, enthält.

Die kosmetischen Mittel der vorliegenden Erfindung können zur gleichzeitigen temporären Farb- und Formveränderung von keratinischen Fasern, insbesondere Haaren verwendet werden. Zudem können diese Mittel zur temporären Farbveränderung der Haut eingesetzt werden. Hierzu können sie in üblichen Formen, beispielsweise als Gel, Spray, Schaum oder Wachs konfektioniert sein. Bevorzugt ist die Konfektionierung als Spray. Derartige Sprays können in Form von Aerosolen und Non-Aerosolen vorliegen und aus dem Fachmann bekannten Behältern versprüht werden. Sind die Mittel als Aerosole konfektioniert, ist zusätzlich mindestens ein Treibmittel enthalten. Erfindungsgemäß geeignete Treibmittel sind beispielsweise ausgewählt aus NzO, Dimethylether, COz, Luft, Alkanen mit 3 bis 5 Kohlenstoffatomen, wie Propan, n-Butan, iso-Butan, n-Pentan und iso-Pentan, sowie deren Mischungen. Ganz besonders bevorzugt wird Dimethylether als Treibmittel eingesetzt. Die vorliegende Erfindung umfasst aber ausdrücklich auch die Mitverwendung von Treibmitteln vom Typ der Fluorchlorkohlenwasserstoffe, insbesondere aber der Fluorkohlenwasserstoffe. Diese Treibmittel sind bevorzugt in einer Gesamtmenge von 30 bis 70 Gew.-%, vorzugsweise von 35 bis 75 Gew.-%, insbesondere von 40 bis 60 Gew.-%, bezogen auf das Gesamtgewicht des kosmetischen Mittels, enthalten.

Die aus dem Stand der Technik bekannten Mascara-Produkte enthalten in aller Regel Fettstoffe. Diese Fettstoffe können ebenfalls einen Film auf den keratinischen Oberflächen bilden, welcher die Pigmente nach der Anwendung vor dem Abrieb oder dem Abwaschen durch Wasser schützt. Der wesentliche Nachteil der Fettstoffe ist jedoch, dass sie auf der keratinischen Oberfläche eine wenig vorteilhafte Haptik erzeugen, welche sich insbesondere in einem Gefühl der Härte und des fettigen Gefühls äußert. Die keratinische Oberfläche wirkt beschwert und vermittelt auch visuell einen fettigen Eindruck.

Es ist daher erfindungsgemäß bevorzugt, wenn die kosmetischen Mittel lediglich einen geringen Anteil an Fettstoffen enthalten. Bevorzugte Ausführungsformen des ersten Erfindungsgegenstands sind daher dadurch gekennzeichnet, dass das kosmetische Mittel Fettstoffe in einer Gesamtmenge von 0 bis 2,5 Gew.-%, vorzugsweise von 0 bis 1,5 Gew.-%, bevorzugt von 0 bis 0,5 Gew.-%, weiter bevorzugt von 0 bis 0,1 Gew.-%, insbesondere von 0 Gew.-%, bezogen auf das Gesamtgewicht des kosmetischen Mittels, enthält. Unter "Fettstoffen" werden im Sinne der Erfindung organische Verbindungen mit einer Löslichkeit in Wasser bei Raumtemperatur (22 °C) und atmosphärischem Druck (760 mm Hg) von weniger als 1 Gew.-%, bevorzugt von weniger als 0,1 Gew.-% verstanden. Unter die Definition der Fettbestandteile fallen explizit nur ungeladene (d.h. nichtionische) Verbindungen. Geladene Verbindungen wie beispielsweise Fettsäuren und ihre Salze werden nicht als Fettbestandteil verstanden. Fettstoffe im Sinne der vorliegenden Erfindung besitzen mindestens eine gesättigte oder ungesättigte Alkylgruppe mit mindestens 12 C-Atomen. Enthalten die Fettstoffe eine ungesättigte Alkylgruppe, so kann diese eine oder mehrere Doppelbindungen besitzen. Das Molgewicht der Fettbestandteile liegt bei maximal 5000 g/mol, bevorzugt bei maximal 2500 g/mol und besonders bevorzugt bei maximal 1000 g/mol. Bei den Fettbestandteilen handelt es sich weder um polyoxyalkylierte noch um polyglycerylierte Verbindungen. Daher fallen Fettalkohole oder Fettsäuren, welche mit mindestens zwei Oxyalkylgruppen bzw. mit mindestens zwei Glycerineinheiten verestert oder verethert sind, nicht unter die Definition der Fettstoffe.

Fettstoffe, welche bevorzugt in einer Gesamtmenge von höchstens 2,5 Gew.-% enthalten sind, sind ausgewählt aus der Gruppe der (i) C₁₂-C₃₀-Fettalkohole; (ii) C₁₂-C₃₀-Fettsäuretriglyceride; (iii) Diester aus einem Äquivalent Ethylenglycol (1,2-Ethandiol) mit zwei Äquivalenten Fettsäure (Ethylenglycol-Difettsäureester); (iv) Wachse; (v) Kohlenwasserstoffe mit mindestens 12 C-Atomen; sowie (vi) deren Mischungen. Bei C₁₂-C₃₀-Fettalkoholen handelt es sich um gesättigte, ein- oder mehrfach ungesättigte, lineare oder verzweigte Fettalkohole mit 12 bis 30 C-Atomen. Beispiele für deartige C₁₂-C₃₀-Fettalkohole sind Dodecan-1-ol (Dodecylalkohol, Laurylalkohol), Tetradecan-1-ol (Tetradecylalkohol, Myristylalkohol), Hexadecan-1-ol (Hexadecylalkohol, Cetylalkohol, Palmitylalkohol), Octadecan-1-ol (Octadecylalkohol, Stearylalkohol), Arachylalkohol (Eicosan-1-ol), Heneicosylalkohol (Heneicosan-1-ol) und/oder Behenylalkohol (Docosan-1-ol). Beispiele für verzweigte Fettalkohole sind 2-Octyl-dodecanol, 2-Hexyl-Dodecanol und/oder 2-Butyl-dodecanol. Unter C₁₂-C₃₀-Fettsäuretriglyceriden werden Mono-, Di- und Triester des dreiwertigen Alkohols Glycerin mit drei Äquivalenten Fettsäure verstanden. Dabei können sowohl struktur- gleiche als auch unterschiedliche Fettsäuren innerhalb eines Triglyceridmoleküls an den Esterbildungen beteiligt sein. Unter Wachsen werden die Ester von C₁₂-C₃₀-Fettsäuren mit C₁₂-C₃₀- Fettalkoholen verstanden. Erfindungsgemäß werden unter Kohlenwasserstoffen Verbindungen verstanden, welche ausschließlich aus den Atomen Kohlenstoff und Wasserstoff bestehen.

Beispiele für Kohlenwasserstoffe sind Mineralöle, flüssige Paraffinöle (z.B. Paraffinium Liquidum oder Paraffinum Perliquidum), Isoparaffinöle, halbfeste Paraffinöle, Paraffinwachse, Hartparaffin (Paraffinum Solidum), Vaseline und Polydecene. Silikone sind von der Definition der Fettstoffe hingegen nicht umfasst.

Erfindungsgemäß bevorzugt sind die nicht oder in den zuvor angeführten Mengenbereichen enthaltenen Fettstoffe daher aus der Gruppe der C₁₂-C₃₀-Fettalkohole, der C₁₂-C₃₀- Fettsäuretriglyceride, C₁₂-C₃₀-Fettsäurediglyceride, der C₁₂-C₃₀-Fettsäuremonoglyceride, der Ethylenglycol-Difettsäureester, der Wachse, der Kohlenwasserstoffe sowie deren Mischungen.

Durch den Einsatz eines speziellen anionischen Polymers in Verbindung mit einem bestimmten Alkohol wird ein gleichmäßiger wasserbeständiger Pigment-haltiger Film gebildet. Dieser Film ist gegenüber äußeren Umwelteinflüssen, wie Wasser, Schweiß und Abreibung, äußerst stabil, lässt sich jedoch durch einmalige Anwendung eines tensidhaltigen Reinigungsmittels rückstandlos von der keratinischen Oberfläche entfernen. Zudem führt der Einsatz dieses Polymers zu einem hohen Halt und einer hohen Flexibilität des gebildeten Polymerfilms. Bei Auftragen diese Mittel auf die Haare wird daher neben einer temporären Farbveränderung gleichzeitig ein hoher und feuchtigkeitsbeständiger Halt erzielt. Darüber hinaus weisen diese Mittel gute kosmetische Eigenschaften nach deren Anwendung auf der Haut sowie den Haaren auf.

Neben den zuvor beschriebenen Komponenten können die erfindungsgemäßen kosmetischen Mittel weitere Inhaltsstoffe enthalten. Zur Gruppe dieser weiteren Inhaltsstoffe zählen insbesondere kosmetisch wirksame Hilfs- und Zusatzstoffe, wie Tenside, Pflegestoffe, Verdicker und pH- Stellmittel.

Die erfindungsgemäßen Mittel können zusätzlich mindestens ein nichtionisches Tensid enthalten. Geeignete nichtionische Tenside sind Alkylpolyglykoside sowie Alkylenoxid-Anlagerungsprodukte an Fettalkohole und Fettsäuren mit jeweils 2 bis 30 Mol Ethylenoxid pro Mol Fettalkohol bzw. Fettsäure erwiesen. Zubereitungen mit guten Eigenschaften werden ebenfalls erhalten, wenn sie als nichtionische Tenside Fettsäureester von ethoxyliertem Glycerin enthalten, die mit mindestens 2 Mol Ethylenoxid umgesetzt wurden. Die nichtionischen Tenside werden in einer Gesamtmenge von 0,1 bis 45 Gew.-%, bevorzugt 1 bis 30 Gew.-% und ganz besonders bevorzugt von 1 bis 15 Gew.-% - bezogen auf das Gesamtgewicht des Mittels - eingesetzt.

Die erfindungsgemäßen Mittel können zusätzlich mindestens ein kationisches Tensid enthalten. Unter kationischen Tensiden werden Tenside, also grenzflächenaktive Verbindungen, mit jeweils einer oder mehreren positiven Ladungen verstanden. Kationische Tenside enthalten ausschließlich positive Ladungen. Üblicherweise sind diese Tenside aus einem hydrophoben Teil und einer hydrophilen Kopfgruppe aufgebaut, wobei der hydrophobe Teil in der Regel aus einem Kohlenwasserstoff-Gerüst (z.B. bestehend aus einer oder zwei linearen oder verzweigten Alkylketten) besteht, und die positive(n) Ladung(en) in der hydrophilen Kopfgruppe lokalisiert sind. Beispiele für kationische Tenside sind
- quartäre Ammoniumverbindungen, die als hydrophobe Reste ein oder zwei Alkylketten mit einer Kettenlänge von 8 bis 28 C-Atomen tragen können,
- quartäre Phosphoniumsalze, substituiert mit einer oder mehreren Alkylketten mit einer Kettenlänge von 8 bis 28 C-Atomen oder
- tertiäre Sulfonium-Salze.

Weiterhin kann die kationische Ladung auch in Form einer Onium-Struktur Bestandteil eines heterozyklischen Ringes (z.B. eines Imidazoliumringe oder einer Pyridiniumringes) sein. Neben der funktionellen Einheit, welche die kationische Ladung trägt, kann das kationische Tensid auch weitere ungeladene funktionelle Gruppen beinhalten, wie dies beispielsweise bei Esterquats der Fall ist. Die kationischen Tenside werden in einer Gesamtmenge von 0,1 bis 45 Gew.-%, bevorzugt 1 bis 30 Gew.-% und ganz besonders bevorzugt von 1 bis 15 Gew.-% - bezogen auf das Gesamtgewicht des Mittels - eingesetzt.

Der Einsatz von anionischen Tensiden hat sich im Hinblick auf die Abriebfestigkeit der Pigmente auf den Keratinfasern als negativ herausgestellt. Aus diesem Grund ist es bevorzugt, in den erfindungsgemäßen Mitteln keine anionischen Tenside einzusetzen. Als anionische Tenside werden oberflächenaktive Mittel mit ausschließlich anionischen Ladungen (neutralisiert durch ein entsprechendes Gegenkation) bezeichnet. Beispiele für anionische Tenside sind Fettsäuren, Alkylsulfate, Alkylethersulfate und Ethercarbonsäuren mit 12 bis 20 C-Atomen in der Alkylgruppe und bis zu 16 Glykolethergruppen im Molekül.

In einer weiteren bevorzugten Ausführungsform sind erfindungsgemäße Mittel dadurch gekennzeichnet, dass sie anionischen Tenside in einer Gesamtmenge von 0 bis 2,5 Gew.-%, vorzugsweise von 0 bis 1,5 Gew.-%, bevorzugt von 0 bis 0,5 Gew.-%, weiter bevorzugt von 0 bis 0,1 Gew.-%, insbesondere von 0 Gew.-%, jeweils bezogen auf das Gesamtgewicht des kosmetischen Mittels, enthalten.

Die erfindungsgemäßen Mittel können weiterhin mindestens ein zwitterionisches und/oder amphoteres Tensid enthalten. Geeignete zwitterionische Tenside sind Betaine, N-Alkyl-N,N-dimethylammonium-glycinate, N-Acyl-aminopropyl-N,N-dimethylammoniumglycinate, und 2-Alkyl-3-carboxymethyl-3-hydroxyethyl-imidazoline. Ein bevorzugtes zwitterionisches Tensid ist unter der INCI-Bezeichnung Cocamidopropyl Betaine bekannt. Geeignete amphotere Tenside sind N-Alkylglycine, N-Alkylpropionsäuren, N-Alkylaminobuttersäuren, N-Alkyliminodipropionsäuren, N-Hydroxyethyl-N-alkylamidopropylglycine, N-Alkyltaurine, N-Alkylsarcosine, 2-Alkylaminopropionsäuren und Alkylaminoessigsäuren. Besonders bevorzugte amphotere Tenside sind N-Kokosalkylaminopropionat, das Kokosacylaminoethylaminopropionat und C₁₂-C₁₈-Acylsarcosin.

Die amphoteren und/oder zwitterionischen Tenside werden in einer Gesamtmenge von 0,1 bis 45 Gew.-%, bevorzugt 1 bis 30 Gew.-% und ganz besonders bevorzugt von 1 bis 15 Gew.-% - bezogen auf das Gesamtgewicht des Mittels - eingesetzt.

Ein Vorteil der erfindungsgemäßen Mittel besteht darin, dass sie sich in vielfältiger Form konfektionieren lassen. Bei Applikation über einen Schwamm oder über eine kleine Bürste können sehr gleichmäßige Farbeffekte und wasser- sowie reibechte Färbungen erzielt werden. Ebenso ist es jedoch auch möglich, die erfindungsgemäßen Mittel als Spray zu konfektionieren. Insbesondere auch die durch die Spray-Applikation erhaltenen Färbungen zeichnen sich durch eine sehr hohe Gleichmäßigkeit und Wasserbeständigkeit aus.

Abhängig von der gewählten Applikationsform werden die erfindungsgemäßen Mittel auf eine bestimmte Viskosität eingestellt. Dies erfolgt üblicherweise durch den Einsatz eines oder mehrerer Verdicker. Bezüglich dieser Verdickungsmittel bestehen keine prinzipiellen Einschränkungen. Es können sowohl organische als auch rein anorganische Verdickungsmittel zum Einsatz kommen.

Geeignete Verdickungsmittel sind anionische, synthetische Polymere; kationische, synthetische Polymere; natürlich vorkommende Verdickungsmittel, wie nichtionische Guargums, Skleroglucangums oder Xanthangums, Gummi arabicum, Ghatti-Gummi, Karaya-Gummi, Tragant-Gummi, Carrageen-Gummi, Agar-Agar, Johannisbrotkernmehl, Pektine, Alginate, Stärke-Fraktionen und Derivate wie Amylose, Amylopektin und Dextrine, sowie Cellulosederivate, wie beispielsweise Methylcellulose, Carboxyalkylcellulosen und Hydroxyalkylcellulosen; nichtionische, vollsynthetische Polymere, wie Polyvinylalkohol oder Polyvinylpyrrolidinon; sowie anorganische Verdickungsmittel, insbesondere Schichtsilikate wie beispielsweise Bentonit, besonders Smektite, wie Montmorillonit oder Hectorit.

Besonders einfach und reproduzierbar lässt sich die Viskosität der Mittel durch Polysaccharide, insbesondere Polysaccharide aus der Gruppe der Carboxy-C₁-C₆-alkyl-cellulosen, der Hydroxy-C₂-C₈-alkylcellulosen, der Alginsäuren und/oder Xanthan Gum, einstellen.

Durch Variation der eingesetzten Polysaccharidmenge kann das Mittel sowohl als Gel für die Bürsten- bzw. Schwammapplikation oder aber auch als niedrigviskose, sprühbare Lösung konfektioniert werden. Die anderen Rezepturbestandteile bzw. ihre Einsatzmengen müssen hierbei nicht angepasst werden, was insbesondere bei der Produktion der Mittel von Vorteil ist. In einer weiteren besonders bevorzugten Ausführungsform ist ein erfindungsgemäßes Mittel daher dadurch gekennzeichnet, dass es als Verdicker zusätzlich mindestens ein Polysaccharid aus der Gruppe der Carboxy-C₁-C₆-alkyl-cellulosen, der Hydroxy-C₂-C₈-alkylcellulosen, der Alginsäuren und/oder Xanthan Gum enthält.

In einer ganz besonders bevorzugten Ausführungsform ist ein erfindungsgemäßes Mittel dadurch gekennzeichnet, dass es als Verdicker zusätzlich mindestens ein Polysaccharid aus der Gruppe der der Hydroxy-C₂-C₈-alkylcellulosen enthält.

Der oder die Verdicker können in den erfindungsgemäßen Mitteln in einer Gesamtmenge von 0,1 bis 4,5 Gew.-%, bevorzugt von 0,15 bis 3,5 Gew.-% und besonders bevorzugt von 0,2 bis 2,0 Gew.-% - bezogen auf das Gesamtgewicht des Mittels - eingesetzt werden.

Einige der zuvor genannten Verdickungsmittel - beispielsweise Schichtsilikate wie Bentonit, besonders Smektit wie Montmorillonit oder Hectorit, können in den erfindungsgemäßen Mitteln zudem als Stabilisierungsmittel dienen.

In einigen Fällen wurde jedoch beobachtet, dass die üblicherweise kationisch modifizierten Bentonite, Garamite und/oder Laponite mitunter zu Stabilitätsverlusten der erfindungsgemäßen Mittel führen können, da sie in negativer Weise mit den erfindungswesentlichen anionischen Polymeren wechselwirken.

Dem konnte durch die Auswahl spezieller, organisch modifizierter Hectorite entgegengewirkt werden. Diese liegen in einem organischen Lösungsmittel als Gel vor und lassen sich leicht in die erfindungsgemäßen Zusammensetzungen einarbeiten. Sie stabilisieren die erfindungsgemäßen Zusammensetzungen zusätzlich und verhindern die Sedimentation und/oder das Zusammenbacken der Pigmente bei der Lagerung.

Besonders geeignete modifizierte Hectorite sind beispielsweise die unter der Handelsbezeichnung Bentone^{®} Gel IPM V (INCI-Bezeichnung: Isopropyl Myristate, Stearalkonium Hectorite; Propylene Carbonate) von der Firma Elementis erhältlich.

Zur Einstellung des pH-Wertes können die erfindungsgemäßen Mittel ein oder mehrere Alkalisierungsmittel enthalten. Die zur Einstellung der gewünschten pH-Werte erfindungsgemäß verwendbaren Alkalisierungsmittel können aus der Gruppe, die gebildet wird aus Ammoniak, Alkanolaminen, basischen Aminosäuren, sowie anorganischen Alkalisierungsmitteln wie (Erd-)Alkalimetallhydroxiden, (Erd-)Alkalimetallmetasilikaten, (Erd-)Alkalimetallphosphaten und (Erd-)Alkalimetallhydrogenphosphaten, ausgewählt werden. Zur Einstellung des pH-Wertes können die erfindungsgemäßen Mittel ein oder mehrere Säuren aus enthalten. Geeignete Säuren sind beispielsweise organische Säuren wie alpha-Hydroxycarbonsäuren oder anorganische Säuren.

Weiterhin können die Mittel ein oder mehrere nichtionische Polymere enthalten. Geeignete nichtionogene Polymere sind beispielsweise:
- Vinylpyrrolidon/Vinylester-Copolymere,
- Stärke und deren Derivate, insbesondere Stärkeether,
- Schellack
- Polyvinylpyrrolidone.

Ferner können die erfindungsgemäßen Mittel weitere Wirk-, Hilfs- und Zusatzstoffe enthalten, wie beispielsweise lineare kationische Polymere wie quaternisierte Celluloseether, Polysiloxane mit quaternären Gruppen, mit Diethylsulfat quaternierte Dimethylamino-ethylmethacrylat-Vinylpyrrolidinon-Copolymere, Vinylpyrrolidinon-Imidazolinium-methochlorid-Copolymere und quaternierter Polyvinylalkohol; zwitterionische und amphotere Polymere (die von den erfindungsgemäßen Polymeren verschieden sind); anionische Polymere wie beispielsweise Polyacrylsäuren oder vernetzte Polyacrylsäuren; Strukturanten wie Glucose, Maleinsäure und Milchsäure, haarkonditionierende Verbindungen wie Phospholipide, beispielsweise Lecitin und Kephaline; Parfümöle, Dimethylisosorbid und Cyclodextrine; faserstrukturverbessernde Wirkstoffe, insbesondere Mono-, Di- und Oligosaccharide wie beispielsweise Glucose, Galactose, Fructose, Fruchtzucker und Lactose; Farbstoffe zum Anfärben des Mittels; Antischuppenwirkstoffe wie Piroctone Olamine, Zink Omadine und Climbazol; Aminosäuren und Oligopeptide; Proteinhydrolysate auf tierischer und/oder pflanzlicher Basis, sowie in Form ihrer Fettsäure-Kondensationsprodukte oder gegebenenfalls anionisch oder kationisch modifizierten Derivate; Lichtschutzmittel und UV-Blocker; Wirkstoffe wie Panthenol, Pantothensäure, Pantolacton, Allantoin, Pyrrolidinoncarbonsäuren und deren Salze sowie Bisabolol; Polyphenole, insbesondere Hydroxyzimtsäuren, 6,7-Dihydroxycumarine, Hydroxybenzoesäuren, Catechine, Tannine, Leukoanthocyanidine, Anthocyanidine, Flavanone, Flavone und Flavonole; Ceramide oder Pseudoceramide; Vitamine, Provitamine und Vitaminvorstufen; Pflanzenextrakte; Fette und Wachse wie Fettalkohole, Bienenwachs, Montanwachs und Paraffine; Quell- und Penetrationsstoffe wie Glycerin, Propylenglykolmonoethylether, Carbonate, Hydrogencarbonate, Guanidine, Harnstoffe sowie primäre, sekundäre und tertiäre Phosphate; Trübungsmittel wie Latex, Styrol/PVP- und Styrol/Acrylamid-Copolymere sowie PEG-3-distearat; Treibmittel wie Propan-Butan-Gemische, NzO, Dimethylether, COz und Luft.

Die Auswahl dieser weiteren Stoffe wird der Fachmann gemäß der gewünschten Eigenschaften der Mittel treffen. Bezüglich weiterer fakultativer Komponenten sowie der eingesetzten Mengen dieser Komponenten wird ausdrücklich auf die dem Fachmann bekannten einschlägigen Handbücher verwiesen. Die zusätzlichen Wirk- und Hilfsstoffe werden in den erfindungsgemäßen Mitteln bevorzugt in Mengen von jeweils 0,0001 bis 25 Gew.-%, insbesondere von 0,0005 bis 15 Gew.-%, bezogen auf das Gesamtgewicht des kosmetischen Mittels, eingesetzt.

Bei der Anwendung in Form eines Pump-Sprays oder in Form eines Aerosol-Sprays kann der Anwender die erfindungsgemäßen Mittel direkt auf das trockene Haar oder die Haut aufsprühen und auf diesem Wege die gewünschte temporäre Farbänderung erzeugen.

Im Falle der Anwendung auf dem Haar kann der Verwender zunächst - beispielsweise durch Kämmen, Toupieren, oder durch die Anwendung eines Lockenstabs - seine Frisur in Form bringen und dann das erfindungsgemäße Mittel aufsprühen. Ebenfalls möglich ist es, zunächst das erfindungsgemäße Mittel aufzusprühen und danach oder währenddessen die Frisur durch die vorgenannten Methoden in Form zu bringen.

Ein zweiter Gegenstand der vorliegenden Erfindung ist daher ein Verfahren zur temporären Verformung und Färbung keratinischer Fasern, wobei das Verfahren die folgenden Verfahrensschritte umfasst:
a) Bereitstellen eines erfindungsgemäßen kosmetischen Mittels in Form eines Gels, eines Pump-Sprays oder eines Aerosol-Sprays,
b) Applizieren, insbesondere Aufsprühen, des in Schritt a) bereitgestellten kosmetischen Mittels auf die keratinischen Fasern,
c) Verteilen des in Schritt b) applizierten kosmetischen Mittels auf den keratinischen Fasern und Verformung der keratinischen Fasern in die gewünschte Form.

Unter keratinhaltigen Fasern werden prinzipiell alle tierischen Haare, z.B. Wolle, Rosshaar, Angorahaar, Pelze, Federn und daraus gefertigte Produkte oder Textilien verstanden. Vorzugsweise handelt es sich bei den keratinischen Fasern jedoch um menschliche Haare.

Bezüglich weiterer bevorzugter Ausführungsformen des erfindungsgemäßen Verfahrens, insbesondere bezüglich des dort eingesetzten kosmetischen Mittels, gilt mutatis mutandis das zu den erfindungsgemäßen Mitteln Gesagte.

Ein dritter Gegenstand der vorliegenden Erfindung ist ein Verfahren zur temporären Färbung der Haut, wobei das Verfahren die folgenden Verfahrensschritte umfasst:
a) Bereitstellen eines erfindungsgemäßen kosmetischen Mittels in Form eines Gels, eines Pump-Sprays oder eines Aerosol-Sprays,
b) Applizieren, insbesondere Aufsprühen, des in Schritt a) bereitgestellten kosmetischen Mittels auf die Haut,
c) Verteilen des in Schritt b) applizierten kosmetischen Mittels auf der Haut.

Bezüglich weiterer bevorzugter Ausführungsformen des erfindungsgemäßen Verfahrens, insbesondere bezüglich des dort eingesetzten kosmetischen Mittels, gilt mutatis mutandis das zu den erfindungsgemäßen Mitteln Gesagte.

Schließlich ist ein weiterer Gegenstand der vorliegenden Erfindung die Verwendung eines anionischen Polymers, umfassend mindestens eine Struktureinheit der Formel (I) und mindestens eine Struktureinheit der Formel (II) und mindestens eine Struktureinheit der Formel (III) 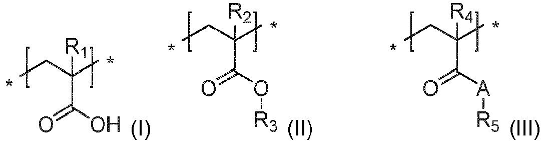 worin
R₁, R₂ und R₄, jeweils unabhängig voneinander, für ein Wasserstoffatom oder eine C₁-C₄-Alkylgruppe stehen,
R₃ für eine verzweigte oder unverzweigte, gesättigte oder ungesättigte C₁-C₁₂-Alkygruppe steht,
R₅ für eine verzweigte oder unverzweigte, gesättigte oder ungesättigte C₆-C₁₄-Alkylgruppe steht und
A für Sauerstoff, Schwefel oder eine NH-Gruppe steht

in Pigment-haltigen kosmetischen Mitteln zur Verbesserung der Wasserbeständigkeit dieser Mittel. Unter Verbesserung der Wasserbeständigkeit wird erfindungsgemäß verstanden, dass der Einsatz dieser Polymere zu einer Verringerung bzw. Vermeidung des Abwaschens der auf der Oberfläche angelagerten Pigmente bei Einsatz von Wasser führt.

Gemäß einer bevorzugten Ausführungsform dieses Erfindungsgegenstands werden anionische Polymere verwendet, welche mindestens eine Struktureinheit der Formel (I) und mindestens eine Struktureinheit der Formel (II) und mindestens eine Struktureinheit der Formel (III) umfassen 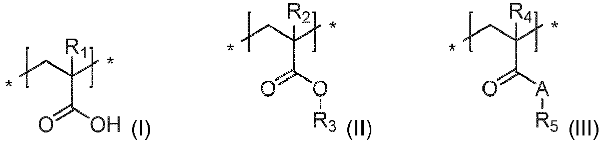 worin
R₁ und R₄ jeweils unabhängig voneinander für Wasserstoff,
R₂ für eine Methylgruppe
R₃ für eine *-CH₂-C(CH₃)₂-Gruppe,
R₅ für eine *-C(CH₃)₂-CH₂-C(CH₃)₃-Gruppe und
A für eine NH-Gruppe steht.

Bezüglich weiterer bevorzugter Ausführungsformen der erfindungsgemäßen Verwendungen gilt mutatis mutandis das zu den erfindungsgemäßen kosmetischen Mitteln sowie den erfindungsgemäßen Verfahren Gesagte.

Die nachfolgenden Beispiele erläutern die vorliegende Erfindung, ohne sie jedoch darauf einzuschränken:

### Beispiele:

### 1. Test der Wasserbeständigkeit

Zur Ermittlung der Wasserbeständigkeit der Färbungen wurden die folgenden Zusammensetzungen hergestellt (alle Angaben in Gewichtsprozent, bezogen auf das Gesamtgewicht des jeweiligen kosmetischen Mittels):

| Rohstoff | V1 | E1 |
|---|---|---|
| Ethanol 99% denat | 80,7 | 80,7 |
| Anionisches Polymer ¹⁾ | - | 1,5 |
| Pigment ²⁾ | 11 | 11 |
| PVP/VA-Copolymer 60/40 W NP | 3,5 | 2,0 |
| Glycerin 99,5% | 2,0 | 2,0 |
| D-Panthenol 75 % | 0,20 | 0,20 |
| Dehyquart A CA ³⁾ | 0,45 | 0,45 |
| Stearamidopropyl Diemthylamine | 1,3 | 1,3 |
| KOH 50% | 0,20 | 0,20 |
| Parfüm | 0,65 | 0,65 |

| | | |
|---|---|---|
| ¹⁾ enthaltend Struktureinheiten der Formeln (I) bis (III) mit R₁, R₄ jeweils = H, R₂ = Methylgruppe, R₃ = *-CH₂-C(CH₃)₂-Gruppe, R₅ = *-C(CH₃)₂-CH₂-C(CH₃)₃-Gruppe, A = NH, ²⁾ Colorona Dark Blue, INCI-Bezeichnung: MICA, TITANIUM DIOXIDE, FERRIC FERROCYANIDE (Merck) ³⁾ INCI-Bezeichnung: AQUA (WATER), CETRIMONIUM CHLORIDE (BASF). | | |

Die kosmetischen Mittel V1 und E1 wurden durch Vermischen der obigen Inhaltsstoffe erhalten und jeweils auf eine Haarsträhne (Kerling 6-0, hellbraun) aufgetragen. Nach dem Trocknen wurden in beiden Fällen eine gleichmäßige dunkelblaue Färbung mit metallischem Schimmer erhalten. Anschließend wurden beide Strähnen mit Wasser gewaschen. Während bei der mit dem erfindungsgemäßen Mittel E1 behandelten Strähne keinerlei Einfluss auf die Färbung festgestellt werden konnte, wurde die mit dem Mittel V1 erzielte Färbung vollständig ausgewaschen. Die mit der Zusammensetzung E1 erzielte Färbung konnte jedoch durch einmalige Wäsche mit einer tensidhaltigen Reinigungszusammensetzung (Shampoo) rückstandslos entfernt werden. Der Einsatz des speziellen anionischen Polymers führt daher im Vergleich zu dem nichtionischen PVP/VA-Polymer zu einer erhöhten Wasserbeständigkeit der temporären Haarfärbung, ohne jedoch die Auswaschbarkeit mittels tensidhaltiger Reinigungsmittel negativ zu beeinflussen.

### 2. Weiteres erfindungsgemäßes Beispiel

| Rohstoff | E1 |
|---|---|
| Ethanol 96% denat | 85,45 |
| Anionisches Polymer ¹⁾ | 0,75 |
| Pigment ⁴⁾ | 7 |
| Glycerin 99,5% | 0,50 |
| D-Panthenol 75 % | 0,20 |
| Parfüm | 0,10 |
| Bentone Gel IPM V ⁵⁾ | 6 |

| | |
|---|---|
| ⁴⁾ INCI-Bezeichnung: MICA, CI 77491 (IRON OXIDES); Merck ⁵⁾ INCI-Bezeichnung: ISOPROPYL MYRISTATE, STEARALKONIUM HECTORITE, PROPYLENE CARBONATE; Elementis | |

## Patentansprüche

1. Kosmetisches Mittel, enthaltend
a) mindestens einen aliphatischen und/oder aromatischen Alkohol mit 2 bis 8 Kohlenstoffatomen,
b) mindestens ein anionisches Polymer, umfassend mindestens eine Struktureinheit der Formel (I) und mindestens eine Struktureinheit der Formel (II) und mindestens eine Struktureinheit der Formel (III) 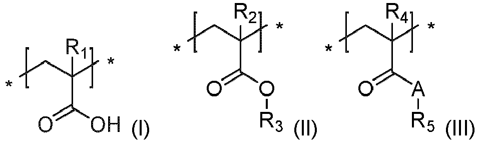 worin
R₁, R₂ und R₄, jeweils unabhängig voneinander, für ein Wasserstoffatom oder eine C₁₋C₄-Alkylgruppe stehen,
R₃ für eine verzweigte oder unverzweigte, gesättigte oder ungesättigte C₁-C₁₂-Alkygruppe steht,
R₅ für eine verzweigte oder unverzweigte, gesättigte oder ungesättigte C₆-C₁₄-Alkylgruppe steht und
A für Sauerstoff, Schwefel oder eine NH-Gruppe steht, und
c) 5,0 bis 20 Gew.-% mindestens eines Pigments, wobei
das kosmetische Mittel als aliphatischen und/oder aromatischen Alkohol mit 2 bis 8 Kohlenstoffatomen a) - bezogen auf sein Gesamtgewicht -
a1) 70 bis 90 Gew.-% Ethanol und
a2) 1,2-Propandiol und/oder Glycerin in einer Gesamtmenge von 1,0 bis 3,5 Gew.-% enthält.

2. Kosmetisches Mittel nach Anspruch 1, **dadurch gekennzeichnet, dass** in den Struktureinheiten der Formeln (I) und (III) die Reste R₁ und R₄, jeweils unabhängig voneinander, für ein Wasserstoffatom stehen und dass in der Struktureinheit der Formel (II) der Rest R₂ für eine Methylgruppe steht.

3. Kosmetisches Mittel nach einem der Ansprüche 1 oder 2, **dadurch gekennzeichnet, dass** in der Struktureinheit der Formel (III) A für eine NH-Gruppe steht.

4. Kosmetisches Mittel nach einem der vorangehenden Ansprüche, **dadurch gekennzeichnet, dass** in der Struktureinheit der Formel (III) der Rest R₅ für eine verzweigte C₆-C₁₀-Alklygruppe, insbesondere für eine *-C(CH₃)₂-CH₂-C(CH₃)₃-Gruppe, steht.

5. Kosmetisches Mittel nach einem der vorangehenden Ansprüche, **dadurch gekennzeichnet, dass** das mindestens eine anionische Polymer a) in einer Gesamtmenge von 0,1 bis 10 Gew.-%, vorzugsweise von 0,5 bis 8,0 Gew.-%, bevorzugt von 1,0 bis 5,5 Gew.-%, insbesondere von 1,0 bis 2,5 Gew.-%, bezogen auf das Gesamtgewicht des kosmetischen Mittels, enthalten ist.

6. Kosmetisches Mittel nach einem der vorangehenden Ansprüche, **dadurch gekennzeichnet, dass** das mindestens eine Pigment ein farbiges Pigment auf Mica- oder Glimmerbasis ist, welches mit einem oder mehreren Metalloxiden aus der Gruppe aus Titandioxid (CI 77891), schwarzem Eisenoxid (CI 77499), gelbem Eisenoxid (CI 77492), rotem und/oder braunem Eisenoxid (CI 77491, CI 77499), Manganviolett (CI 77742), Ultramarine (Natrium-Aluminiumsulfosilikate, CI 77007, Pigment Blue 29), Chromoxidhydrat (CI 77289), Chromoxid (CI 77288) und/oder Eisenblau (Ferric Ferrocyanide, CI 77510) beschichtet ist.

7. Kosmetisches Mittel nach einem der vorangehenden Ansprüche, **dadurch gekennzeichnet, dass** das mindestens eine Pigment in einer Gesamtmenge von 7,0 bis 18 Gew.-%, insbesondere von 8,5 bis 15,5 Gew.-%, jeweils bezogen auf das Gesamtgewicht des kosmetischen Mittels, enthalten ist.

8. Verfahren zur temporären Verformung und Färbung keratinischer Fasern, wobei das Verfahren die folgenden Verfahrensschritte umfasst:
a) Bereitstellen eines kosmetischen Mittels nach einem der Ansprüche 1 bis 7 in Form eines Gels, eines Pump-Sprays oder eines Aerosol-Sprays,
b) Applizieren, insbesondere Aufsprühen, des in Schritt a) bereitgestellten kosmetischen Mittels auf die keratinischen Fasern,
c) Verteilen des in Schritt b) applizierten kosmetischen Mittels auf den keratinischen Fasern und Verformung der keratinischen Fasern in die gewünschte Form.

9. Verfahren zur temporären Färbung der Haut, wobei das Verfahren die folgenden Verfahrensschritte umfasst:
a) Bereitstellen eines kosmetischen Mittels nach einem der Ansprüche 1 bis 7 in Form eines Gels, eines Pump-Sprays oder eines Aerosol-Sprays,
b) Applizieren, insbesondere Aufsprühen, des in Schritt a) bereitgestellten kosmetischen Mittels auf die Haut,
c) Verteilen des in Schritt b) applizierten kosmetischen Mittels auf der Haut.

10. Verwendung eines anionischen Polymers, umfassend mindestens eine Struktureinheit der Formel (I) und mindestens eine Struktureinheit der Formel (II) und mindestens eine Struktureinheit der Formel (III) 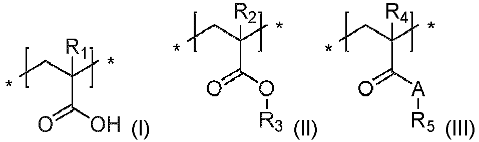 worin
R₁, R₂ und R₄, jeweils unabhängig voneinander, für ein Wasserstoffatom oder eine C₁₋C₄-Alkylgruppe stehen,
R₃ für eine verzweigte oder unverzweigte, gesättigte oder ungesättigte C₁-C₁₂-Alkygruppe steht,
R₅ für eine verzweigte oder unverzweigte, gesättigte oder ungesättigte C₆-C₁₄-Alkylgruppe steht und
A für Sauerstoff, Schwefel oder eine NH-Gruppe steht
in Pigment-haltigen kosmetischen Mitteln, wobei die Mittel - bezogen auf ihr Gesamtgewicht -
- a1) 70 bis 90 Gew.-% Ethanol und
- a2) 1,2-Propandiol und/oder Glycerin in einer Gesamtmenge von 1,0 bis 3,5 Gew.-% und
- 5 - 20 Gew.-% mindestens eines Pigments enthalten,
zur Verbesserung der Wasserbeständigkeit dieser Mittel.

## Claims

1. A cosmetic agent, containing
a) at least one aliphatic and/or aromatic alcohol having 2 to 8 carbon atoms,
b) at least one anionic polymer comprising at least one structural unit of formula (I), and at least one structural unit of formula (II) and at least one structural unit of formula (III), 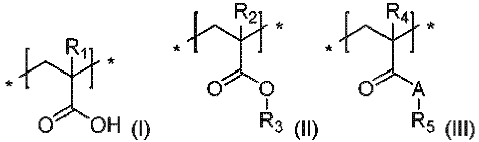 where
R₁, R₂ and R₄ each independently represent a hydrogen atom or a C₁-C₄ alkyl group,
R₃ represents a branched or unbranched, saturated or unsaturated C₁-C₁₂ alkyl group,
R₅ represents a branched or unbranched, saturated or unsaturated C₆-C₁₄ alkyl group and
A represents oxygen, sulfur or an NH group, and
c) 5.0 to 20 wt.% of at least one pigment, wherein
the cosmetic agent contains as aliphatic and/or aromatic alcohol having 2 to 8 carbon atoms a) - based on its total weight -
a1) 70 to 90 wt.% ethanol and
a2) 1,2-propanediol and/or glycerol in a total amount of from 1.0 to 3.5 wt.%.

2. The cosmetic agent according to claim 1, **characterized in that** in the structural units of formulas (I) and (III) the groups R₁and R₄ each independently represent a hydrogen atom and **in that** in the structural unit of formula (II) the group R₂ represents a methyl group.

3. The cosmetic agent according to one of claims 1 or 2,
**characterized in that** in the structural unit of formula (III) A represents an NH group.

4. The cosmetic agent according to one of the preceding claims,
**characterized in that** in the structural unit of formula (III) the group R₅ represents a branched C₆-C₁₀ alkyl group, in particular a *-C(CH₃)₂-CH₂-C(CH₃)₃ group.

5. The cosmetic agent according to one of the preceding claims,
**characterized in that** the at least one anionic polymer a) is contained in a total amount of from 0.1 to 10 wt.%, preferably from 0.5 to 8.0 wt.%, more preferably from 1.0 to 5.5 wt.%, in particular from 1.0 to 2.5 wt.%, based on the total weight of the cosmetic agent.

6. The cosmetic agent according to one of the preceding claims,
**characterized in that** the at least one pigment is a mica-based colored pigment which is coated with one or more metal oxides from the group of titanium dioxide (CI 77891), black iron oxide (CI 77499), yellow iron oxide (CI 77492), red and/or brown iron oxide (CI 77491, Cl 77499), manganese violet (CI 77742), ultramarine (sodium aluminum sulfosilicates, Cl 77007, Pigment Blue 29), chromium oxide hydrate (CI 77289), chromium oxide (CI 77288) and/or iron blue (ferric ferrocyanide, Cl 77510).

7. The cosmetic agent according to one of the preceding claims,
**characterized in that** the at least one pigment is contained in a total amount of 7.0 to 18 wt.%, in particular 8.5 to 15.5 wt.%, in each case based on the total weight of the cosmetic agent.

8. A method for temporarily deforming and dyeing keratin fibers, wherein the method comprises the following method steps:
a) providing a cosmetic agent according to one of claims 1 to 7 in the form of a gel, a pump spray or an aerosol spray,
b) applying, in particular spraying, the cosmetic agent provided in step a) to the keratin fibers,
c) distributing the cosmetic agent applied in step b) to the keratin fibers and deforming the keratin fibers into the desired shape.

9. A method for temporarily dyeing the skin, wherein the method comprises the following method steps:
a) providing a cosmetic agent according to one of claims 1 to 7 in the form of a gel, a pump spray or an aerosol spray,
b) applying, in particular spraying, the cosmetic agent provided in step a) to the skin,
c) distributing the cosmetic agent applied in step b) on the skin.

10. A use of an anionic polymer comprising at least one structural unit of formula (I), at least one structural unit of formula (II) and at least one structural unit of formula (III), 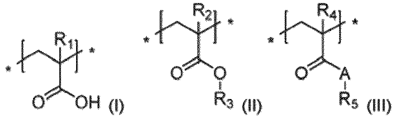 where
R₁, R₂ and R₄ each independently represent a hydrogen atom or a C₁-C₄ alkyl group,
R₃ represents a branched or unbranched, saturated or unsaturated C₁-C₁₂ alkyl group,
R₅ represents a branched or unbranched, saturated or unsaturated C₆-C₁₄ alkyl group and
A represents oxygen, sulfur or an NH group in pigment-containing cosmetic agents, wherein the agents contain, - based on their total weight -
- a1) 70 to 90 wt.% ethanol and
- a2) 1 ,2-propanediol and/or glycerol in a total amount of from 1.0 to 3.5 wt.% and
- 5 - 20 wt.% of at least one pigment
for improving the water resistance of these agents.

## Revendications

1. Agent cosmétique, contenant
(a) au moins un alcool aliphatique et/ou aromatique comportant 2 à 8 atomes de carbone,
b) au moins un polymère anionique, comprenant au moins un motif structural de formule (I) et au moins un motif structural de formule (II) et au moins un motif structural de formule (III) 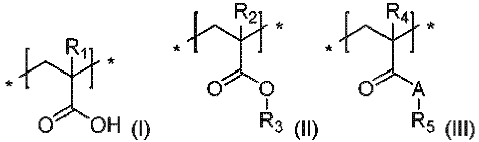 OÙ
R₁, R₂ et R₄ représentent, respectivement indépendamment les uns des autres, un atome d'hydrogène ou un groupe alkyle en C₁-C₄,
R₃ représente un groupe alkyle en C₁-C₁₂ ramifié ou non ramifié, saturé ou insaturé,
R₅ représente un groupe alkyle en C₆-C₁₄ ramifié ou non ramifié, saturé ou insaturé et
A représente l'oxygène, le soufre ou un groupe NH, et
c) 5,0 à 20 % en poids d'au moins un pigment, dans lequel
l'agent cosmétique contient, en tant qu'alcool aliphatique et/ou aromatique comportant 2 à 8 atomes de carbone a), par rapport à son poids total,
a1) 70 à 90 % en poids d'éthanol et
a2) du 1,2-propanediol et/ou du glycérol en une quantité totale de 1,0 à 3,5 % en poids.

2. Agent cosmétique selon la revendication 1, **caractérisé en ce que,** dans les motifs structuraux de formules (I) et (III), les radicaux R₁ et R₄ représentent, respectivement indépendamment l'un de l'autre, un atome d'hydrogène,
**et en ce que**, dans le motif structural de formule (II), le radical R₂ représente un groupe méthyle.

3. Agent cosmétique selon l'une des revendications 1 ou 2,
**caractérisé en ce que**, dans le motif structural de formule (III), A représente un groupe NH.

4. Agent cosmétique selon l'une des revendications précédentes,
**caractérisé en ce que**, dans le motif structural de formule (III), le radical R₅ représente un groupe alkyle en C₆-C₁₀ ramifié, en particulier un groupe *-C(CH₃)₂-CH₂-C(CH₃)₃.

5. Agent cosmétique selon l'une des revendications précédentes,
**caractérisé en ce que** l'au moins un polymère anionique a) est contenu en une quantité totale de 0,1 à 10 % en poids, de préférence de 0,5 à 8,0 % en poids, de manière davantage préférée de 1,0 à 5,5 % en poids, en particulier de 1,0 à 2,5 % en poids, par rapport au poids total de l'agent cosmétique.

6. Agent cosmétique selon l'une des revendications précédentes,
**caractérisé en ce que** l'au moins un pigment est un pigment coloré à base de mica, lequel est recouvert d'un ou de plusieurs oxydes métalliques du groupe constitué de dioxyde de titane (CI 77891), oxyde de fer noir (CI 77499), oxyde de fer jaune (CI 77492), oxyde de fer rouge et/ou brun (Cl 77491, Cl 77499), violet de manganèse (CI 77742), bleu d'outremer (sulfosilicates de sodium et d'aluminium, CI 77007, Pigment Blue 29), oxyde de chrome hydraté (Cl 77289), oxyde de chrome (Cl 77288) et/ou bleu de Prusse (ferrocyanure ferrique, CI 77510).

7. Agent cosmétique selon l'une des revendications précédentes,
**caractérisé en ce que** l'au moins un pigment est contenu en une quantité totale de 7,0 à 18 % en poids, en particulier de 8,5 à 15,5 % en poids, respectivement par rapport au poids total de l'agent cosmétique.

8. Procédé permettant la mise en forme et la coloration temporaires de fibres kératiniques, dans lequel le procédé comprend les étapes de procédé suivantes :
a) fourniture d'un agent cosmétique selon l'une des revendications 1 à 7 sous la forme d'un gel, d'un spray à pompe ou d'un spray à aérosol,
b) application, en particulier par pulvérisation, de l'agent cosmétique fourni à l'étape a) sur les fibres kératiniques,
c) répartition de l'agent cosmétique appliqué à l'étape b) sur les fibres kératiniques et mise en forme des fibres kératiniques dans la forme souhaitée.

9. Procédé permettant la coloration temporaire de la peau, dans lequel le procédé comprend les étapes de procédé suivantes :
a) fourniture d'un agent cosmétique selon l'une des revendications 1 à 7 sous la forme d'un gel, d'un spray à pompe ou d'un spray à aérosol,
b) application, en particulier par pulvérisation, de l'agent cosmétique fourni à l'étape a) sur la peau,
c) répartition de l'agent cosmétique appliqué à l'étape b) sur la peau.

10. Utilisation d'un polymère anionique, comprenant au moins un motif structural de formule (I) et au moins un motif structural de formule (II) et au moins un motif structural de formule (III) 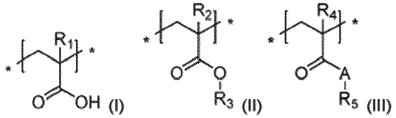
où R₁, R₂ et R₄ représentent, respectivement indépendamment les uns des autres, un atome d'hydrogène ou un groupe alkyle en C₁-C₄,
R₃ représente un groupe alkyle en C₁-C₁₂ ramifié ou non ramifié, saturé ou insaturé,
R₅ représente un groupe alkyle en C₆-C₁₄ ramifié ou non ramifié, saturé ou insaturé et
A représente l'oxygène, le soufre ou un groupe NH
dans des agents cosmétiques contenant des pigments, dans lequel les agents contiennent, par rapport à leur poids total,
- a1) 70 à 90 % en poids d'éthanol et
- a2) du 1,2-propanediol et/ou du glycérol en une quantité totale de 1,0 à 3,5 % en poids et
- 5 à 20 % en poids d'au moins un pigment,
pour l'amélioration de la résistance à l'eau desdits agents.
